# EUROPEAN PATENT APPLICATION

(11) **EP 4 030 168 A1**
(43) Date of publication of application: **20.07.2022**
(21) Application number: 20863832.0
(22) Date of filing: 31.08.2020
(51) Int. Cl.: G01N 33/569, G01N 33/68

(54) **LUNG DISEASE DIAGNOSTIC METHOD BASED ON ANTIBODIES AGAINST MICROORGANISM-DERIVED VESICLES**

(30) Priority: 10.09.2019 KR 20190111920; 11.02.2020 KR 20200016035
(71) Applicant: MD Healthcare Inc., Seoul 03923 (KR)
(72) Inventor: KIM, Yoon-Keun, Paju-Si, Gyeonggi-do 10908 (KR)
(74) Representative: Dehns
(86) International application number: PCT/KR2020/011631
(87) International publication number: WO 2021/049798

(57) **Abstract**

The present invention relates to a method for diagnosing a lung disease based on antibodies against microorganism-derived vesicles and, more particularly, to a method for diagnosing a lung disease by performing bacterial metagenome analysis using samples derived from a subject to analyze an increase or decrease in IgG, IgG1 and/or IgG4 levels in extracellular vesicles derived from specific bacteria.

## Description

### [Technical Field]

The present invention relates to a method for diagnosis of lung disease based on antibodies for microbial-derived vesicles and the like.

This application claims priority to and the benefit of Korean Patent Application Nos. 10-2019-0111920 and 10-2020-0016035 filed in the Korean Intellectual Property Office on September 10, 2019 and February 11, 2020, respectively, and all the contents disclosed in the specification and drawings of the applications are incorporated in this application.

### [Background Art]

Interest in the indoor environment is increasing due to the importance of the indoor environment and its overall impact on health. Most people spend 87% and 5 to 6% of their time on average indoors and in vehicles, respectively. Indoor dust including PM10, PM2.5, nanoparticles and house dust mites that affect peoples's health is generated. Biological components in indoor air may cause immune dysfunction and inflammation, thereby leading to inflammatory pulmonary diseases such as asthma and chronic obstructive pulmonary disease (COPD). Asthma, chronic obstructive pulmonary disease and lung cancer are major diseases which cause high burden and mortality worldwide. Further, it has been suggested that chronic obstructive pulmonary disease and lung cancer share a common cause of smoking. However, some patients with chronic obstructive pulmonary disease or lung cancer have never been exposed to smoking, and in this case, the chronic obstructive pulmonary disease or lung cancer may be caused by materials other than cigarette smoke. These materials may be due to occupational exposure to gas or dust, exposure to biomass fuel combustion, unknown causative organisms and the like.

Chronic obstructive airway diseases are lung diseases characterized by respiratory difficulty due to chronic airway obstruction and may be broadly classified into asthma, which is characterized by reversible airway obstruction, and chronic obstructive pulmonary disease (COPD), which is characterized by irreversible airway obstruction. According to data of the National Statistical Office in 2012, the number of annual deaths due to respiratory diseases has been steadily increasing since 2006, and among these, death due to COPD is known to be the only cause of death which is on the rise, among the 10 leading causes of death, and the 2013 WHO report has shown that COPD-induced death is the fourth leading cause of death worldwide.

COPD is a disease in which chronic inflammation of the lungs causes the occurrence of chronic bronchitis, chronic bronchiolitis, and emphyema, resulting in irreversible airway obstruction. With regard to the causative factors of COPD, it is known that smoking or chemicals such as air pollutants and biological factors derived from viruses, bacteria, or the like are important. COPD is a disease that is pathologically characterized by neutrophilic inflammation, and this is immunologically characterized by Th17 cell-induced hypersensitivity. Meanwhile, asthma is a disease characterized by airway hypersensitivity to non-specific stimuli and chronic inflammatory responses, and is characterized by Th2 cell-induced hypersensitivity due to allergens such as protein antigens derived from house dust mites and the like and eosinophilic inflammation occurring due to this.

Lung cancer is a malignant tumor originating from the lungs and is one of the leading causes of death in Korea with a 5-year survival rate of less than 50% despite the development of modern medicine. Lung cancer is broadly classified into small cell lung cancer and non-small cell lung cancer according to histological type, and since the aforementioned small cell lung cancer has the characteristic of being clearly distinguished from different types of lung cancer in terms of treatment method and prognosis, the result of histological examination is very important for determining the treatment policy for lung cancer.

Although lung cancer exhibits symptoms such as a cough, hemoptysis, chest pain, and dyspnea, lung cancer is often already in an advanced stage by the time when the symptoms appear in many cases, and even when lung cancer is progressing, a case in which a patient is asymptomatic often occurs, and only 5 to 15% of patients with lung cancer are diagnosed when they are asymptomatic, and most of the patients are diagnosed with lung cancer after symptoms appear. To date, studies have been actively conducted on early diagnosis of lung cancer using computed tomography (CT), but its effectiveness has not yet been proven. Therefore, there is an urgent need for the development of a method capable of enhancing treatment efficiency by diagnosing lung cancer early, and prior to this, since it is very important to differentiate a response method for early diagnosis and treatment by making it possible to predict the onset of lung cancer in advance, there is a need for research and development on this.

Meanwhile, It is known that the number of microorganisms symbiotically living in the human body is 100 trillion which is 10 times the number of human cells, and the number of genes of microorganisms exceeds 100 times the number of human genes. A microbiota or microbiome is a microbial community that includes bacteria, archaea, and eukaryotes present in a given habitat. The intestinal microbiota is known to play a vital role in human's physiological phenomena and significantly affect human health and diseases through interactions with human cells. Bacteria coexisting in human bodies secrete nanometer-sized vesicles to exchange information about genes, proteins, and the like with other cells. The mucous membranes form a physical barrier membrane that does not allow particles with the size of 200 nm or more to pass therethrough, and thus bacteria symbiotically living in the mucous membranes are unable to pass therethrough, but bacteria-derived extracellular vesicles have a size of approximately 100 nm or less and thus relatively freely pass through the mucous membranes and are absorbed into the human body.

Metagenomics, also called environmental genomics, may be analytics for metagenomic data obtained from samples collected from the environment (Korean Patent Publication No. 2011-0073049). Recently, the bacterial composition of human microbiota has been listed using a method based on 16s ribosomal RNA (16s rRNA) base sequences, and 16s rDNA base sequences, which are genes of 16s ribosomal RNA, are analyzed using a next generation sequencing (NGS) platform. However, in the onset of asthma, chronic obstructive airway diseases, and lung cancer, identification of causative factors of chronic obstructive airway diseases such as asthma, COPD, and lung cancer and the like through metagenomic analysis of bacteria-derived vesicles isolated from a human-derived substance, such as blood and the like, and a method of diagnosing a risk of developing asthma, chronic obstructive airway diseases, and lung cancer, have never been reported.

### [Disclosure]

### [Technical Problem]

Therefore, an object of the present invention is to provide a method for providing information for diagnosing a lung disease, the method comprising the following steps:
(a) obtaining a sample from a subject;
(b) measuring protein levels using antibodies against one or more proteins selected from the group consisting of extracellular vesicle IgG, extracellular vesicle IgG1 and extracellular vesicle IgG4 for the obtained sample; and
(c) comparing the protein levels with those of a control.

Another object of the present invention is to provide a composition for diagnosing a lung disease, comprising an agent which measures the levels of one or more proteins selected from the group consisting of extracellular vesicle IgG, extracellular vesicle IgG1 and extracellular vesicle IgG4.

Still another object of the present invention is to provide a kit for diagnosing a lung disease, comprising the diagnostic composition.

Yet another object of the present invention is to provide a method for screening a therapeutic agent for a lung disease, the method comprising: administering a candidate material for treating a lung disease to a subject, and then measuring protein levels using antibodies against one or more proteins selected from the group consisting of extracellular vesicle IgG, extracellular vesicle IgG1 and extracellular vesicle IgG4 for a sample obtained from the subject.

However, the technical goals of the present invention are not limited to the aforementioned goals, and other unmentioned technical goals will be clearly understood by those of ordinary skill in the art from the following description.

### [Technical Solution]

To achieve the above-described object of the present invention, there is provided a method for providing information for diagnosing a lung disease, the method comprising the following steps:
(a) obtaining a sample from a subject;
(b) measuring protein levels using antibodies against one or more proteins selected from the group consisting of extracellular vesicle IgG, extracellular vesicle IgG1 and extracellular vesicle IgG4 for the obtained sample; and
(c) comparing the protein levels with those of a control.

The present invention also provides a method for providing information for predicting a risk for a lung disease, the method comprising the following steps:
(a) obtaining a sample from a subject;
(b) measuring protein levels using antibodies against one or more proteins selected from the group consisting of extracellular vesicle IgG, extracellular vesicle IgG1 and extracellular vesicle IgG4 for the obtained sample; and
(c) comparing the protein levels with those of a control.

The present invention also provides a method for diagnosing a lung disease, the method comprising the following steps:
(a) obtaining a sample from a subject;
(b) measuring protein levels using antibodies against one or more proteins selected from the group consisting of extracellular vesicle IgG, extracellular vesicle IgG1 and extracellular vesicle IgG4 for the obtained sample; and
(c) comparing the protein levels with those of a control.

The present invention also provides a composition for diagnosing a lung disease, comprising an agent which measures the levels of one or more proteins selected from the group consisting of extracellular vesicle IgG, extracellular vesicle IgG1 and extracellular vesicle IgG4.

The present invention also provides a kit for diagnosing a lung disease, comprising the composition.

The present invention also provides a method for diagnosing a lung disease using the composition.

The present invention also provides use of the composition for diagnosing a lung disease.

The present invention also provides a method for screening a therapeutic agent for a lung disease, the method comprising: administering a candidate material for treating a lung disease to a subject, and then measuring protein levels using antibodies against one or more proteins selected from the group consisting of extracellular vesicle IgG, extracellular vesicle IgG1 and extracellular vesicle IgG4 for a sample obtained from the subject.

In one embodiment of the present invention, the sample may be blood, and the blood may be whole blood, serum, plasma, or blood mononuclear cells, but is not limited thereto.

In another embodiment of the present invention, the lung disease may be one or more selected from the group consisting of asthma, chronic obstructive pulmonary disease, lung cancer, pneumonia, emphysema, chronic bronchitis, and pulmonary hypertension, but is not limited thereto.

In still another embodiment of the present invention, the agent may be an antibody that specifically binds to the protein, but is not limited thereto.

In still another embodiment of the present invention, the extracellular vesicle in Step (b) may be an extracellular vesicle derived from bacteria, but is not limited thereto.

In still another embodiment of the present invention, Step (b) may further comprise measuring protein levels using antibodies against the extracellular vesicle IgG2 or extracellular vesicle IgG3 protein, but is not limited thereto.

In still another embodiment of the present invention, Step (c) may predict that the risk of developing a lung disease is high when the levels of IgG of extracellular vesicles derived from one or more genus bacteria selected from the group consisting of *Staphylococcus, Acinetobacter, Enterobacter,* and *Pseudomonas* are increased, but is not limited thereto.

In still another embodiment of the present invention, Step (c) may predict that the risk of developing a lung disease is high when the levels of IgG1 of extracellular vesicles derived from one or more genus bacteria selected from the group consisting of *Staphylococcus, Acinetobacter, Enterobacter,* and *Pseudomonas* are increased, but is not limited thereto.

In still another embodiment of the present invention, Step (c) may predict that the risk of developing a lung disease is high when the levels of IgG4 of extracellular vesicles derived from one or more genus bacteria selected from the group consisting of *Staphylococcus, Acinetobacter, Enterobacter,* and *Pseudomonas* are increased, but is not limited thereto.

In yet another embodiment of the present invention, when the lung disease is asthma, Step (b) may measure protein levels using an antibody against a protein of IgG of extracellular vesicles derived from bacteria of one or more genera selected from the group consisting of *Staphylococcus, Enterobacter* and *Pseudomonas;* and an antibody against a protein of IgG1 of extracellular vesicles derived from the bacteria of the genus *Acinetobacter,* but is not limited thereto.

In still another embodiment of the present invention, when the lung disease is COPD, Step (b) may measure protein levels using an antibody against a protein of IgG of extracellular vesicles derived from bacteria of one or more genera selected from the group consisting of *Staphylococcus, Acinetobacter, Enterobacter* and *Pseudomonas;* and an antibody against a protein of IgG1 of extracellular vesicles derived from the bacteria of the genus *Acinetobacter,* but is not limited thereto.

In still another embodiment of the present invention, when the lung disease is lung cancer, Step (b) may measure protein levels using an antibody against a protein of IgG of extracellular vesicles derived from bacteria of one or more genera selected from the group consisting of *Staphylococcus, Enterobacter* and *Pseudomonas;* and an antibody against a protein of IgG1 of extracellular vesicles derived from the bacteria of the genus *Acinetobacter,* but is not limited thereto.

### [Advantageous Effects]

Extracellular vesicles secreted from bacteria in the environment are absorbed into the human body and thus may directly affect the inflammatory response, and it is difficult to diagnose chronic obstructive airway disease such as asthma, COPD, lung cancer and the like early before symptoms occur, and thus efficient treatment therefor is difficult. Thus, according to the present invention, a risk of developing lung diseases can be predicted through antibody-based IgG, IgG1, and IgG4 level analysis of bacteria-derived extracellular vesicles using a human body-derived sample, and thus the onset of lung diseases can be delayed or prevented through appropriate management by early diagnosis and prediction of a risk group for lung diseases, and, even after lung diseases occur, early diagnosis therefor can be implemented, thereby lowering the incidence rate of lung diseases and increasing therapeutic effects.

### [Description of Drawings]

FIG. 1A illustrates the results showing the distribution of bacteria in indoor dust.
FIG. 1B illustrates the results showing the distribution of extracellular vesicles (EVs) derived from bacteria in indoor dust.
FIG. 1C illustrates the results showing the diversity of bacteria and EV distribution in indoor dust of apartment houses and hospitals with the Shannon index.
FIG. 2A illustrates the results showing the distribution of bacteria in dust of apartment houses with the Taxon analysis.
FIG. 2B illustrates the results showing the distribution of bacteria in dust of apartment houses with the OTU analysis.

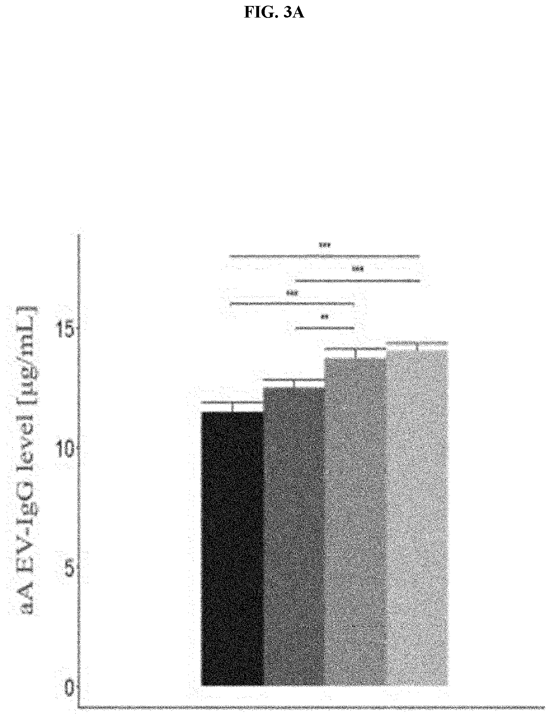

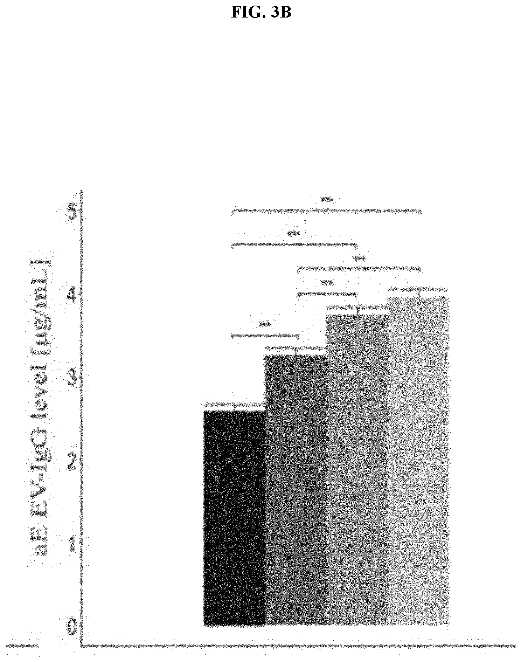

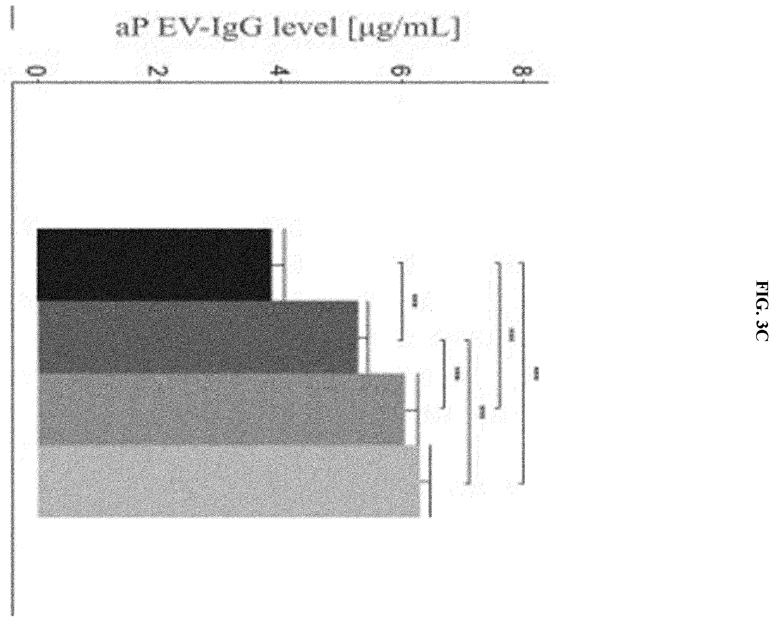

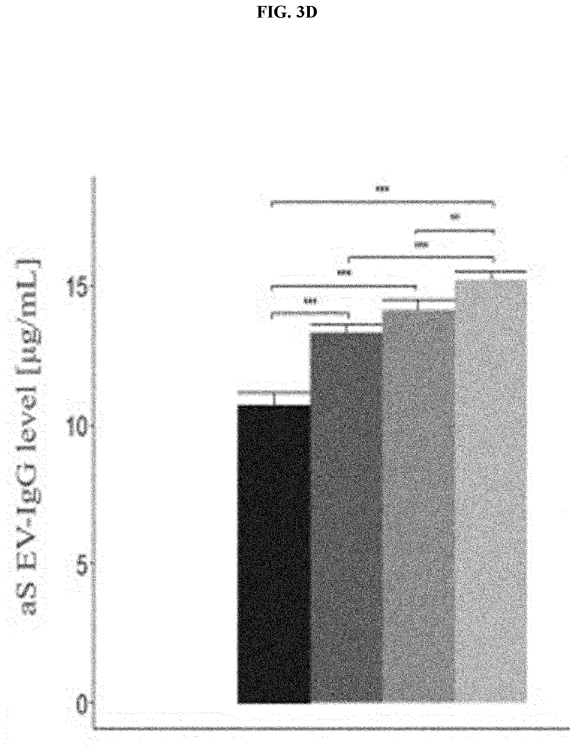

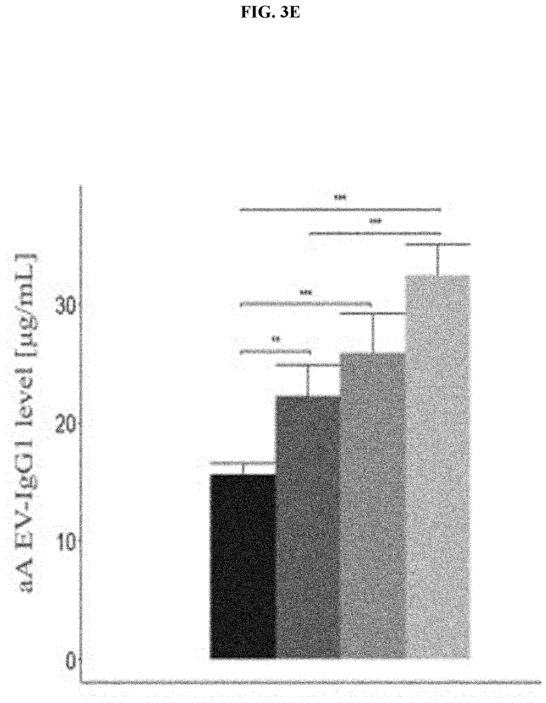

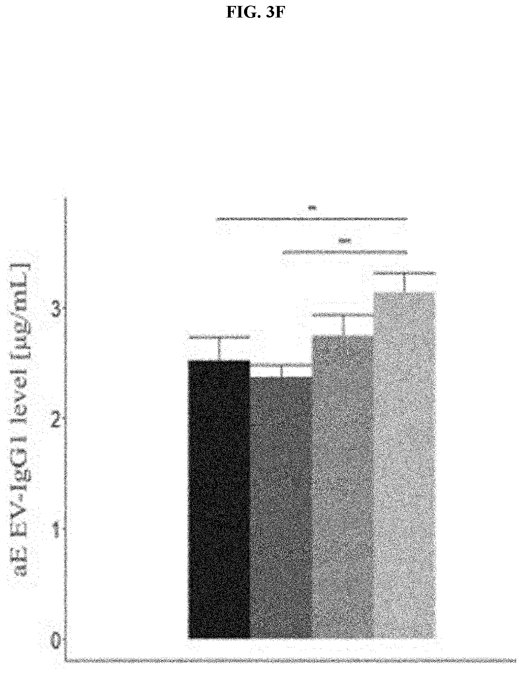

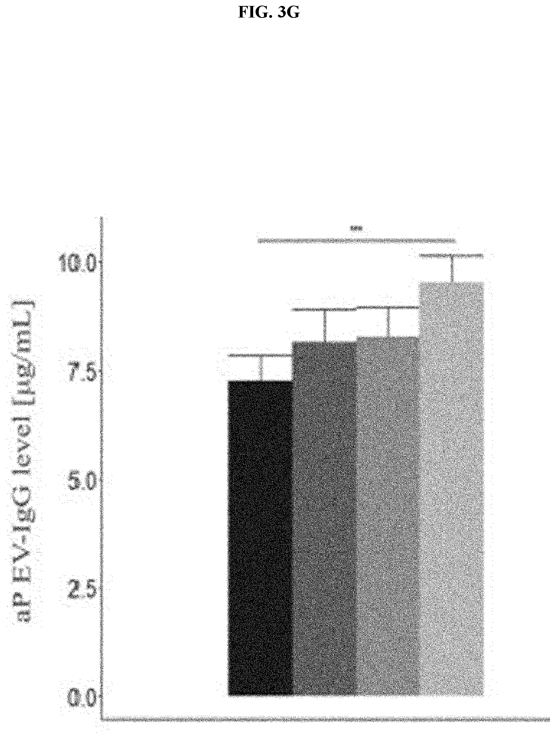

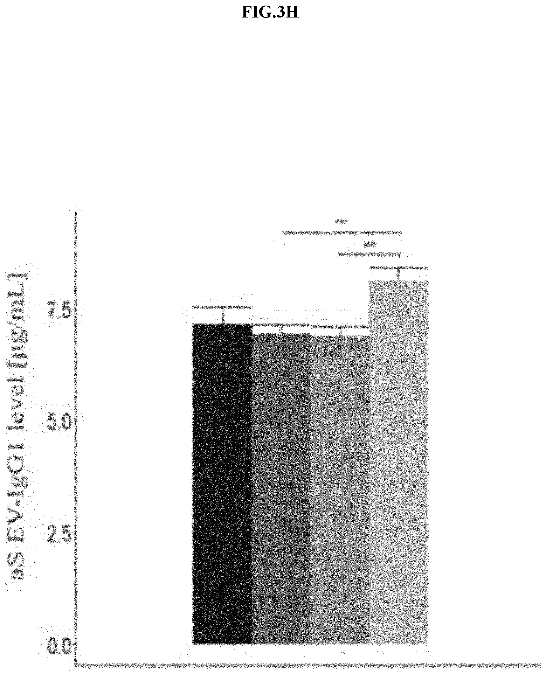

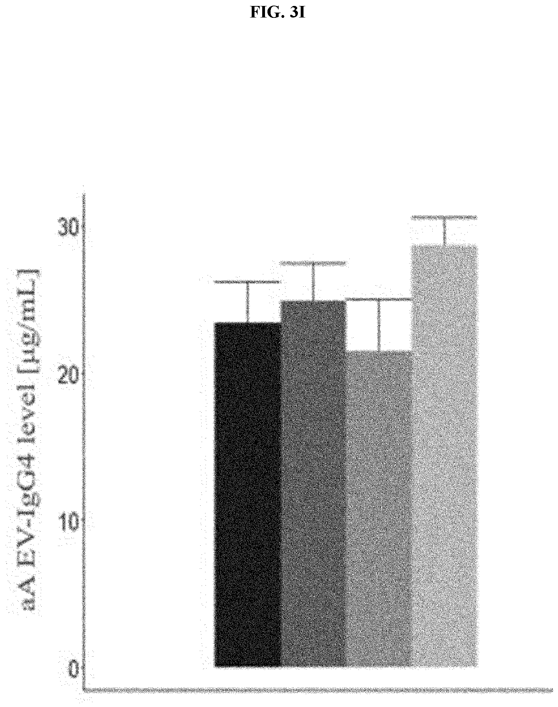

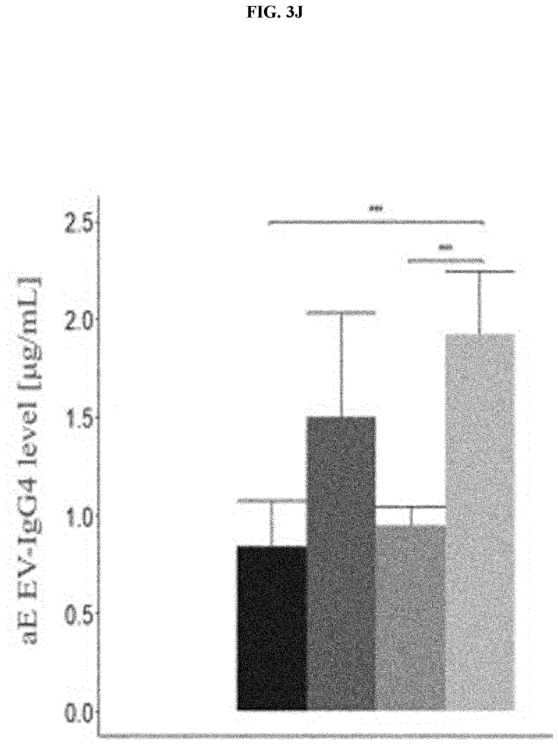

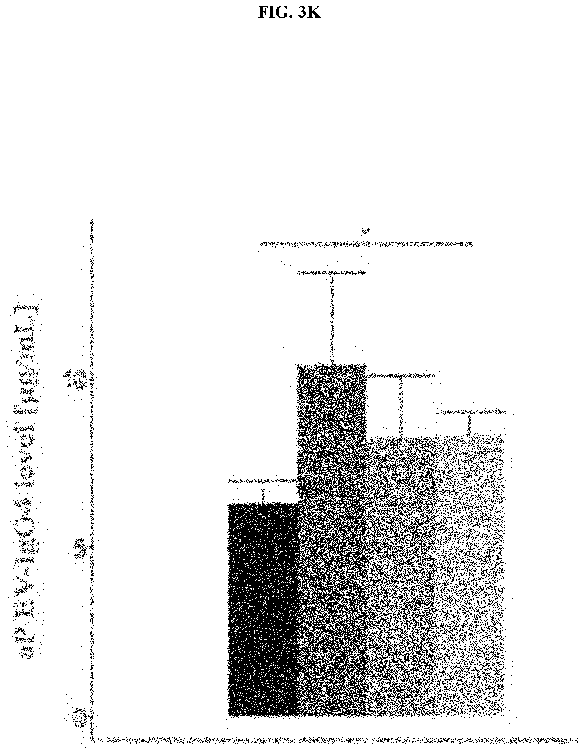

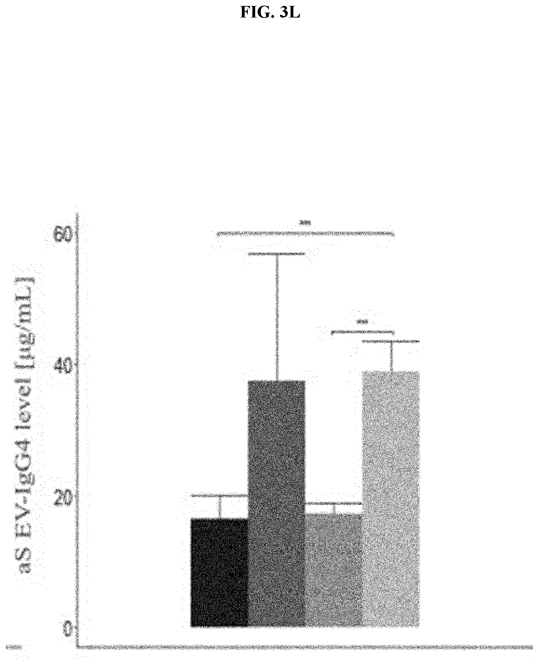

FIGS. 3A to 3L illustrate the difference in sensitivity of IgG, IgG1 and IgG4 against *A. baumannii, E. cloacae, P. aeruginosa*, and *S. aureus* microbial EVs, FIG. 3A illustrates the sensitivity of IgG against *A. baumannii* EV, FIG. 3B illustrates the sensitivity of IgG against *E. cloacae* EV, FIG. 3C illustrates the sensitivity of IgG against *P. aeruginosa*, FIG. 3D illustrates the sensitivity of IgG against *S. aureus* EV, FIG. 3E illustrates the sensitivity of IgG1 against *A. baumannii* EV, FIG. 3F illustrates the sensitivity of IgG1 against *E. cloacae* EV, FIG. 3G illustrates the sensitivity of IgG1 against *P. aeruginosa* EVs, FIG. 3H illustrates the sensitivity of IgG1 against *S. aureus* EV, FIG. 3I illustrates the sensitivity of IgG4 against *A. baumannii* EV, FIG. 3J illustrates the sensitivity of IgG4 against *E*. *cloacae* EV, FIG. 3K illustrates the sensitivity of IgG4 against *P. aeruginosa* EV, and FIG. 3L illustrates the sensitivity of IgG4 against *S. aureus* (aE : anti-E. *cloacae,* aP : anti-*P*. *aeruginosa,* aS : anti-*S*. *aureus,* aA : anti-*A*. *baumannii,* ^{∗∗} = P <0.05, ^{∗∗∗} = P <0.01, from the left to right, normal persons, asthma, COPD, and lung cancer in this order).
FIG. 4A illustrates the results obtained by performing a logistic regression analysis in an asthma diagnostic model (ADM) (Red: ADMi, Green: ADMii).
FIG. 4B illustrates the results obtained by performing a logistic regression analysis in a COPD diagnostic model (CDM) (Red: CDMi, Green: CDMii, Blue: CDMiii, Yellow: CDMiv).
FIG. 4C illustrates the results obtained by performing a logistic regression analysis in a lung cancer diagnostic model (LDM) for a normal control (Red: LDMi, Green: LDMii, Blue: LDMiii, Yellow: LDMiv).
FIG. 4D illustrates the results obtained by performing a logistic regression analysis in a lung cancer diagnostic model (LDMC) for patients with COPD (Red: LDMCi, Green: LDMCii, Blue: LDMCiii, Yellow: LDMCiv).
FIGS. 5A to 5D illustrate the results of confirming the most abundant species in the genus *Acinetobacter,* FIG. 5A illustrates the analysis of the genus *Acinetobacter,* and FIGS. 5B, 5C, and 5D illustrate the results of confirming *Acinetobacter baumanii, Acinetobacter schindleri,* and *Acinetobacter lwoffii*, respectively.
FIG. 6 illustrates the results of confirming that the most abundant species in the genus *Pseudomonas* is *Pseudomonas aeruginosa*.
FIG. 7 illustrates the results of confirming that the most abundant species in the genus *Enterobacteriaceae* (f) is *Enterobacter cloacae.*

### [Modes of the Invention]

The present invention relates to a lung disease by measuring the level of a specific protein using antibodies, and the present inventors measured the protein levels of extracellular vesicle IgG, IgG1 and IgG4 from bacteria-derived extracellular vesicles using samples derived from a subject.

Therefore, the present invention provides a method for providing information for diagnosing a lung disease, the method comprising the following steps:
(a) obtaining a sample from a subject;
(b) measuring protein levels using antibodies against one or more proteins selected from the group consisting of extracellular vesicle IgG, extracellular vesicle IgG1 and extracellular vesicle IgG4 for the obtained sample; and
(c) comparing the protein levels with those of a control.

In the present invention, the Step (c) may predict that the risk of developing a lung disease is high when the levels of IgG of extracellular vesicles derived from one or more genus bacteria selected from the group consisting of *Staphylococcus, Acinetobacter, Enterobacter,* and *Pseudomonas* are increased, but is not limited thereto.

In the present invention, the Step (c) may predict that the risk of developing a lung disease is high when the levels of IgG1 of extracellular vesicles derived from one or more genus bacteria selected from the group consisting of *Staphylococcus, Acinetobacter, Enterobacter,* and *Pseudomonas* are increased, but is not limited thereto.

In the present invention, the Step (c) may predict that the risk of developing a lung disease is high when the levels of IgG4 of extracellular vesicles derived from one or more genus bacteria selected from the group consisting of *Staphylococcus, Acinetobacter, Enterobacter,* and *Pseudomonas* are increased, but is not limited thereto.

In the present invention, the genus Staphylococcus may be, for example, *Staphylococcus aureus,* but is not limited thereto.

In the present invention, the genus *Acinetobacter* may be, for example, *Acinetobacter baumannii,* but is not limited thereto.

In the present invention, the genus *Enterobacter* may be, for example, *Enterobacter cloacae,* but is not limited thereto.

In the present invention, the genus *Pseudomonas* may be, for example, *Pseudomonas aeruginosa*, but is not limited thereto.

As used herein, the term "lung disease" refers to a concept comprising one or more selected from the group consisting of asthma, chronic obstructive pulmonary disease, lung cancer, pneumonia, emphysema, chronic bronchitis, and pulmonary hypertension, but is not limited thereto.

The term "COPD" as used herein is a concept comprising chronic bronchitis, chronic bronchiolitis, and emphyema, but the present invention is not limited thereto.

As used herein, the term "diagnosis of a lung disease" refers to determining whether a patient is likely to develop a lung disease, has a relatively high probability of developing a lung disease, or has already developed a lung disease, determining the susceptibility of a subject to a lung disease, determining whether a subject currently has a lung disease, determining the prognosis of a subject with a lung disease, or therametrics (for example, monitoring the condition of a subject to provide information on therapeutic efficacy). The method of the present invention may be used to delay the onset of lung disease through special and appropriate care for a specific patient, which is a patient having a high risk for lung disease or prevent the onset of lung disease. In addition, the method may be clinically used to determine treatment by selecting the most appropriate treatment method through early diagnosis of lung disease.

In the present invention, the method for providing information for diagnosing asthma may comprise measuring protein levels using antibodies against a protein of IgG of extracellular vesicles derived from bacteria of one or more genera selected from the group consisting of *Staphylococcus, Enterobacter,* and *Pseudomonas;* and an antibody against a protein of IgG1 of extracellular vesicles derived from the bacteria of the genus *Acinetobacter,* but is not limited thereto.

In the present invention, the method for providing information for diagnosing COPD may comprise measuring protein levels using antibodies against a protein of IgG of extracellular vesicles derived from bacteria of one or more genera selected from the group consisting of *Staphylococcus, Acinetobacter, Enterobacter,* and *Pseudomonas;* and an antibody against a protein of IgG1 of extracellular vesicles derived from the bacteria of the genus *Acinetobacter,* but is not limited thereto.

In the present invention, the method for providing information for diagnosing lung cancer may comprise measuring protein levels using antibodies against a protein of IgG of extracellular vesicles derived from bacteria of one or more genera selected from the group consisting of *Staphylococcus, Enterobacter,* and *Pseudomonas;* and an antibody against a protein of IgG1 of extracellular vesicles derived from the bacteria of the genus *Acinetobacter,* but is not limited thereto.

In the present invention, the method for providing information for diagnosing lung cancer may comprise measuring protein levels using antibodies against a protein of IgG of extracellular vesicles derived from bacteria of one or more genera selected from the group consisting of *Staphylococcus, Acinetobacter, Enterobacter,* and *Pseudomonas;* an antibody against a protein of IgG1 of extracellular vesicles derived from the bacteria of one or more genera selected from the group consisting of *Enterobacter, Pseudomonas* and *Acinetobacter;* and an antibody against a protein of IgG4 of extracellular vesicles derived from the bacteria of one or more genera selected from the group consisting of *Staphylococcus, Enterobacter, and Acinetobacter;* but is not limited thereto.

In the present invention, the subject may be a potential patient, but is not limited thereto.

In the present invention, the subject with lung cancer may be a potential patient or a COPD patient, but is not limited thereto.

In the present invention, the method for providing information for diagnosing lung cancer may be performed by obtaining samples from a COPD patient, and may comprise measuring protein levels using an antibody against an IgG4 protein of extracellular vesicles derived from the bacteria of the genus *Staphylococcus;* and an antibody against an IgG4 protein of extracellular vesicles derived from the bacteria of the genus *Enterobacter,* during diagnosis of lung cancer for the patient with COPD, but is not limited thereto.

In the present invention, the method for providing information for diagnosing lung cancer of COPD patient may comprise measuring protein levels using antibodies against a protein of IgG, IgG1 and IgG4 of extracellular vesicles derived from the bacteria of the genus *Staphylococcus*; and an antibody against a protein of IgG4 of extracellular vesicles derived from the bacteria of the genus *Enterobacter,* but is not limited thereto.

The term "metagenome" as used herein refers to the total of genomes comprising all viruses, bacteria, fungi, and the like in isolated regions such as soil, the intestines of animals, and the like, and is mainly used as a concept of genomes that explains identification of many microorganisms at once using a sequencer to analyze non-cultured microorganisms. In particular, a metagenome does not refer to a genome of one species, but refers to a mixture of genomes, comprising genomes of all species of an environmental unit. This term originates from the view that, when defining one species in a process in which biology is advanced into omics, various species as well as existing one species functionally interact with each other to form a complete species. Technically, it is the subject of techniques that analyzes all DNAs and RNAs regardless of species using rapid sequencing to identify all species in one environment and verify interactions and metabolism. In the present invention, bacterial metagenomic analysis is performed using bacteria-derived extracellular vesicles isolated from, for example, serum.

As used herein, the term 'expression' refers to production of a protein or nucleic acid.

As used herein, the term 'protein' is interchangeably with 'polypeptide' or 'peptide', and refers to, for example, a polymer of amino acid residues as generally found in proteins in a natural state.

As used herein, the term 'detection' refers to a meaning comprising all of measuring and confirming whether a target material (a marker protein and extracellular vesicle IgG, IgG1 and IgG4 in the present invention) is present (expressed), or measuring and confirming a change in presence level (expression level) of a target material. In the same context, the measuring of the expression level of the protein in the present invention means measuring whether the protein is expressed (that is, measuring the presence or absence of expression), or measuring the level of qualitative and quantitative changes in the protein. The measurement may be performed without limitation by comprising all qualitative (analytical) and quantitative methods. The types of qualitative and quantitative methods in the measurement of the protein levels are well known in the art, and include the experimental methods described herein. Specific methods for comparing protein levels for each method are well known in the art. Therefore, detection of the extracellular vesicle IgG, IgG1 and IgG4 proteins means including detecting whether extracellular vesicle IgG, IgG1 and IgG4 proteins are present, or confirming an increase (upregulation) in expression amount of the protein.

In the present invention, the meaning of 'increase in expression (or high expression)' of a protein means that the protein that was not expressed is expressed (that is, the protein that was not detected is detected) or the protein is relatively overexpressed (that is, the detected amount is increased) compared to the normal level. A person skilled in the art can understand that the meaning of the opposite term has the opposite meaning according to the aforementioned definition.

In the present invention, the method of detecting a protein is not particularly limited as long as the detection is based on a method for measuring the expression level of a protein known in the art, but as an example, it is possible to detect or measure the protein using an antibody that specifically binds to the protein. Specifically, the detection of the protein in the present invention is not limited to, but may be based on any one selected from the group consisting of, for example, western blot, enzyme linked immunosorbent assay (ELISA), radioimmunoassay, radioimmunodiffusion, Ouchterlony immunodiffusion, rocket immunoelectrophoresis, immunostaining (including immunohistochemical staining, immunocytochemical staining, immunofluorescence staining, and the like), immunoprecipitation assay, complement fixation assay, fluorescence activated cell sorter (FACS), surface plasmon resonance (SPR) or protein chips. In addition, the measurement method is understood in accordance with the following description for an agent for measuring the extracellular vesicle IgG, IgG1 and IgG4 expression levels and a kit including the same provided in the present invention.

In the present invention, the sample may be blood, and the blood may be whole blood, serum, plasma, or blood mononuclear cells, but is not limited thereto.

Furthermore, the method may be relatively performed with a sample of a negative control (particularly, a normal control). Therefore, the method may further comprise detecting extracellular vesicle IgG, IgG1 and/or IgG4 (measuring the expression level of extracellular vesicle IgG, IgG1 and/or IgG4), and then comparing the protein levels of extracellular vesicle IgG, IgG1 and/or IgG4 detected from samples taken from a potential patient with those of samples of a negative control. As used herein, the term normal control refers to all of a sample taken from a normal site in a sample (that is, the same individual as the patient to be tested in Step (a)) of a potential patient to be tested, or a sample taken from another normal individual (a subject without any lung disease). In this case, when the protein levels of extracellular vesicle IgG, IgG1 and/or IgG4 detected from samples taken from the potential patient are higher than the levels of a negative control (particularly, a normal control), it may be determined that the potential patient is a patient with a lung disease.

Therefore, the method of the present invention may be understood as a method for improving accuracy (AUC), and when extracellular vesicle IgG, IgG1 and/or IgG4 are/is used as markers, the accuracy may exhibit a level of 70% to 100%, preferably 72% to 98%, and more preferably 75% to 95%. Specific numerical values of the levels include all range values bounded by two numbers selected from the group consisting of 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% and 100%. In one embodiment of the present invention, boundary values of 80% and 90% may be specifically selected from the numerical range, and accordingly, it is obvious to those skilled in the art that all values within a range of 80% to 90% are intended in the present invention. In another embodiment of the present invention, it is possible to indicate an accuracy of preferably 80% to 88%, and the value is not limited thereto.

In order to improve accuracy, the present invention may set the presence or absence of smoking as a covariate, but is not limited thereto. Therefore, Step (c) may compare the protein levels with those of the control depending on the presence or absence of smoking, but is not limited thereto.

In an example of the present invention, it was confirmed that when the presence or absence of smoking was set as a covariate, the accuracy of each of the asthma, lung cancer, and COPD diagnostic models was remarkably improved (see Example 4 of the present invention).

The present invention also provides a composition for diagnosing a lung disease, including an agent which measures the levels of one or more proteins selected from the group consisting of extracellular vesicle IgG, extracellular vesicle IgG1 and extracellular vesicle IgG4, and a kit for diagnosing a lung disease, comprising the composition.

The type of agent which measures the levels of extracellular vesicle IgG, extracellular vesicle IgG1 and/or extracellular vesicle IgG4 proteins is not particularly limited as long as it is known to be capable of being used for the measurement of the expression level of the protein in the art, but the agent may be preferably an antibody that specifically binds to extracellular vesicle IgG, extracellular vesicle IgG1 and/or extracellular vesicle IgG4 proteins.

As used herein, the term 'antibody' refers to an immunoglobulin that specifically binds to an antigenic site. More specifically, the antibody refers to a glycoprotein including at least two heavy (H) chains and two light (L) chains linked by disulfide bonds. Each heavy chain consists of a heavy chain variable region (hereinafter, abbreviated as HCVR or VH) and a heavy chain constant region. The heavy chain constant region consists of three domains, CH1, CH2 and CH3. Each light chain consists of a light chain variable region (hereinafter, abbreviated as LCVR or VL) and a light chain constant region. The light chain constant region consists of one domain, CL. The VH and VL regions may be further subdivided into hypervariable regions (referred to as complementarity determining regions (CDRs)), interspersed with regions that are more conserved, referred to as framework regions (FRs). Each VH and VL consists of 3 CDRs and 4 FRs, arranged from the amino terminal to the carboxyl terminal in the following order: FR1, CDR1, FR2, CDR2, FR3, CDR3, FR4. The variable regions of heavy chains and light chains contain a binding domain that interacts with an antigen. The constant region of the antibody may include various cells (for example, effector cells) of the immune system and a first component (C1q) of a traditional complement system to mediate immunoglobulin binding to host tissues or factors.

In the present invention, the anti-extracellular vesicle IgG, anti-extracellular vesicle IgG1 and/or anti-extracellular vesicle IgG4 antibodies/antibody are/is antibodies/an antibody specifically binding only to extracellular vesicle IgG, extracellular vesicle IgG1 and/or extracellular vesicle IgG4 proteins/protein without reacting with other types of proteins other than extracellular vesicle IgG, extracellular vesicle IgG1 and/or extracellular vesicle IgG4. The anti-extracellular vesicle IgG, anti-extracellular vesicle IgG1 and anti-extracellular vesicle IgG4 antibodies may be prepared by a typical method in the art, such as cloning extracellular vesicle IgG, extracellular vesicle IgG1 and extracellular vesicle IgG4 genes into expression vectors to obtain a protein encoded by the genes and obtaining an antibody produced by injecting the obtained protein into an animal. Antibodies against the extracellular vesicle IgG, extracellular vesicle IgG1 and extracellular vesicle IgG4 may be manufactured by extracellular vesicle IgG, extracellular vesicle IgG1 and extracellular vesicle IgG4 full-length sequence proteins, or extracellular vesicle IgG, extracellular vesicle IgG1 and/or extracellular vesicle IgG4 protein-specific antibodies may also be prepared using fragments of extracellular vesicle IgG, extracellular vesicle IgG1 and/or extracellular vesicle IgG4 proteins comprising extracellular vesicle IgG, extracellular vesicle IgG1 and/or extracellular vesicle IgG4 antigenic sites. The specific sequence and form of the antibody of the present invention are not particularly limited, and include a polyclonal antibody or a monoclonal antibody. Further, the type of antibody provided as an immunoglobulin is not particularly limited, and as an example, may be selected from the group consisting of IgG, IgA, IgM, IgE and IgD, and may be preferably an IgG antibody. Furthermore, when the antibody of the present invention can specifically bind to extracellular vesicle IgG, extracellular vesicle IgG1 and/or extracellular vesicle IgG4 proteins/protein, the antibody also includes a special antibody such as a humanized antibody or a chimeric antibody. In addition, when the antibody of the present invention has antigen-antibody binding (reaction), a part of the whole antibody is also included in the antibody of the present invention, and all types of immunoglobulin antibodies that specifically bind to extracellular vesicle IgG, extracellular vesicle IgG1 and extracellular vesicle IgG4 are included. For example, the antibody of the present invention may be not only a complete form of antibody with two full-length light chains and two full-length heavy chains, but also a functional fragment of the antibody molecule, that is, a form such as Fab, F(ab'), F(ab')2, Fv, a diabody, and scFv.

A fragment antigen-binding (Fab) is an antigen-binding fragment of an antibody, and consists of one variable domain and one constant domain of each of the heavy chain and the light chain. F(ab')2 is a fragment produced by hydrolyzing an antibody with pepsin and two Fabs are linked by a disulfide bond at a heavy chain hinge. F(ab') is a monomeric antibody fragment in which a heavy chain hinge is added to a Fab separated by reducing the disulfide bond of the F(ab')2 fragment. A variable fragment (Fv) is an antibody fragment consisting only of the variable regions of each of the heavy and light chains. A single chain variable fragment (scFv) is a recombinant antibody fragment in which a heavy chain variable region (VH) and a light chain variable region (VL) are linked by a flexible peptide linker. A diabody refers to a fragment in which the VH and VL of scFv are linked by a very short linker, such that they cannot be bonded to each other and form a dimer by binding to the VL and VH of another scFv of the same type, respectively, and for the purpose of the present invention, the antibody fragment is not limited in structure or form as long as it maintains the binding specificity for extracellular vesicle IgG, extracellular vesicle IgG1 and extracellular vesicle IgG4 proteins derived from a human.

In the present invention, detection agents (representatively an antibody and a functional fragment thereof, and the like) may be generally labeled with a detectable moiety for the 'detection' thereof. For example, the detection agents may be labeled with a radioisotope or fluorescent label using the technique described in the literature [Current Protocols in Immunology, Volumes 1 and 2, 1991, Coligen et al., Ed. Wiley- Interscience, New York, N. Y., Pubs]. Alternatively, various enzyme-substrate labels can be used, and examples of the enzymatic label include a luciferase such as Drosophila luciferase and bacterial luciferase (US Patent No. 4,737,456), luciferin, 2,3-dihydrophthalazinediones, malate dehydrogenase, urase, a peroxidase such as horseradish peroxidase (HRPO), alkaline phosphatase, β-galactosidase, glucoamylase, lysozyme, a saccharide oxidase (for example, glucose oxidase, galactose oxidase, and glucose-6-phosphate dehydrogenase), a heterocyclic oxidase (for example, uricase and xanthine oxidase), lactoperoxidase, microperoxidase, and the like. Techniques for conjugating enzymes to antibodies are described in, for example, the literature [O'Sullivan et al., 1981, Methods for the Preparation of Enzyme-Antibody Conjugates for use in Enzyme Immunoassay, in Methods in Enzym. (J. Langone & H. Van Vunakis, eds.), Academic press, N. Y., 73: 147-166]. A label may be directly or indirectly conjugated to an antibody using various known techniques. For example, the antibody may be conjugated to biotin, and any labels belonging to the aforementioned three broad categories may be conjugated to avidin and vice versa. Biotin selectively binds to avidin, and accordingly, the label may be conjugated to the antibody in such an indirect manner. Alternatively, to achieve indirect conjugation of a label to an antibody, the antibody may be conjugated to a small hapten (for example, digoxin), and one of the different types of labels mentioned above may be conjugated to an anti-hapten antibody (for example, an anti-digoxin antibody). Therefore, indirect conjugation of a label to an antibody may be achieved.

The composition of the present invention may further include an excipient, a carrier, a labeling material capable of generating detectable signals, a solubilizer, a detergent, and the like, but is not limited thereto. When the labeling material is an enzyme, it is possible to include a substrate capable of measuring enzyme activity and a reaction terminator together. The carrier may be, but is not limited to, a soluble carrier, for example, a physiologically acceptable buffer known in the art, for example, PBS; an insoluble carrier, for example, polystyrene, polyethylene, polypropylene, polyester, polyacrylonitrile, a fluororesin, crosslinked dextran, polysaccharide, a polymer such as magnetic fine particles obtained by plating metal on latex, different types of paper, glass, metal, agarose, and a combination thereof.

The kit for diagnosing a lung disease may selectively include not only an antibody that specifically recognizes the protein, but also one or more types of other constituent compositions, solutions, or devices suitable for an analysis method, in order to measure the protein levels of extraceullular vesicle IgG, extracellular vesicle IgG1 and extracellular vesicle IgG4.

The kit of the present invention may comprise instructions comprising one or more selected from the group consisting of a description on how to use the kit, a lung disease diagnostic indicator and a treatment for a lung disease.

As a specific aspect, the kit may be a diagnostic kit characterized by comprising known essential elements and subsidiary elements needed for performing western blot, ELISA, radioimmunoassay, radioimmunodiffusion, Ouchterlony immunodiffusion, rocket immunoelectrophoresis, immunostaining, immunoprecipitation assay, complement fixation assay, FACS, SPR or a protein chip method, but is not limited thereto.

As an example, the kit comprises specific antibodies against proteins of extracellular vesicle IgG, extracellular vesicle IgG1 and/or extracellular vesicle IgG4. The antibody is an antibody having high specificity and affinity for a target marker protein and having almost no (substantially no) cross-reactivity with other proteins, and is a monoclonal antibody, a polyclonal antibody or a recombinant antibody. Further, the kit may further comprise an antibody specific for any control protein. The kit provided in the kit may be labeled with a moiety that is detectable by itself, and is as described above. In addition, the kit may comprise a separate reagent capable of detecting a bound antibody, for example, a labeled secondary antibody, chromophores, an enzyme (as a form conjugated to the antibody) and a substrate thereof, or other materials capable of binding to the antibody, and the like. Furthermore, the kit of the present invention may comprise a washing solution or eluent capable of removing an excess chromogenic substrate, unbound proteins, and the like and retaining only a protein marker binding to an antibody.

In the examples of the present invention, ELISA was performed using antibodies against extracellular vesicles derived from bacteria in the blood of normal persons, patients with asthma, patients with COPD and patients with lung cancer, and as a result, the expression levels of extracellular vesicle IgG, IgG1 and IgG4 were confirmed.

The present invention confirmed through the example results as described above that the contents of extracellular vesicle IgG, IgG1 and IgG4 derived from bacteria in the blood of normal persons, patients with asthma, patients with COPD, and patients with lung cancer were significantly changed by performing ELISA on extracellular vesicles derived from bacteria, isolated from blood (see the examples of the present invention).

The present invention also provides a method for screening a therapeutic agent for a lung disease, the method comprising: administering a candidate material for treating a lung disease to a subject, and then measuring protein levels using antibodies against one or more proteins selected from the group consisting of extracellular vesicle IgG, extracellular vesicle IgG1 and extracellular vesicle IgG4 for a sample obtained from the subject.

Specifically, the method for screening a therapeutic agent for a lung disease may be usefully used for screening a therapeutic agent for a lung disease as a method for comparing an increase or decrease in expression of one or more proteins selected from the group consisting of extracellular vesicle IgG, extracellular vesicle IgG1 and extracellular vesicle IgG4, in the presence and absence of a candidate material for treating a lung disease. A material that indirectly or directly reduces the expression levels of one or more proteins selected from the group consisting of extracellular vesicle IgG, extracellular vesicle IgG1 and extracellular vesicle IgG4 may be selected as a therapeutic agent for kidney transplant rejection.

That is, after the expression level of the marker of the present invention is measured from a biological sample obtained from a mammal other than a human in the absence of a candidate material for treating a lung disease, and the expression level of the marker of the present invention is also measured in the presence of the candidate material for treating a lung disease to compare both the expression levels, it is possible to select a material which reduces the expression level of the marker of the present invention in the presence of the candidate material for treating a lung disease compared to that of the marker in the absence of the candidate material for treating a lung disease.

In the present invention, the term "subject" refers to mammals such as human or non-human primates, mice, rats, dogs, cats, horses, and cattle.

Throughout the specification of the present application, when one part "comprises" one constituent element, unless otherwise specifically described, this does not mean that another constituent element is excluded, but means that another constituent element may be further comprised. Throughout the specification of the present application, a term of a degree, such as "about" or "substantially", is used in a corresponding numerical value or used as a meaning close to the numerical value when a natural manufacturing and a material tolerance error are presented in a described meaning, and is used to prevent an unconscientious infringer from illegally using disclosed contents comprising a numerical value illustrated as being accurate or absolute in order to help understanding of the present invention. Throughout the specification of the present application, terms, such as a "step (of performing or doing)~ " or a "step of ~" does not mean a "step for ~".

Throughout the specification of the present application, the term "combination(s) thereof' comprised in the Markush type expression means a mixture or combination of at least one selected from the group consisting of constituent elements described in the Markush type expression, and means comprising at least one selected from the group consisting of the constituent elements.

Throughout the specification of the present application, the description "A and/or B" means "A or B, or A and B".

Hereinafter, the present invention will be described with reference to exemplary examples to aid in understanding of the present invention. However, these examples are provided only for illustrative purposes and are not intended to limit the scope of the present invention.

### [Experiment preparation]

### 1. Indoor dust sampling

Dust samples from apartment house mattresses and hospital mattresses in Seoul were filtered with gauze, and centrifuged twice at 10,000 g for 15 minutes. Sampling was performed in apartment houses in March, June, September and December, and in hospitals in July and February.

### 2. Isolation of extracellular vesicles (EVs) and DNA extraction

The dust samples were cultured in PBS at 4°C for 12 hours with stirring, and then filtered with gauze, and centrifuged twice at 10,000 g for 15 minutes. After centrifugation, pellets consisted of bacteria, and for supernatant fractionation, the samples were filtered with a 0.45 µm vacuum filter, and concentrated using a QuixStand Benchtop System (Amersham Biosciences, Little Chalfont, UK) having a 100 kDa hollow fiber membrane. Additional filtration with a 0.22 µm vacuum filter was performed to remove the remaining cells. EVs were centrifuged at 150,000 g at 4°C for 3 hours using a 45 Ti rotor (Beckman Instruments, Fullerton, CA, USA). Thereafter, EVs were diluted with phosphate buffered saline (PBS) and stored at 80°C.

### 3. Bacterial metagenomic analysis of human stool samples

Bacterial genomic DNA was amplified with 16S_V3_F and 16S_V4_R primers specific for the V3-V4 hypervariable region of the 16S rDNA gene, and the primer sequences are shown in Table 1 below. A library was prepared using PCR products according to the MiSeq System guide (Illumina, USA) and quantified using QIAxpert (QIAGEN, Germany). After each amplicon was quantified, the amplicon was set at an equimolar ratio and pooled to determine sequences by MiSeq (Illumina, USA) according to the manufacturer's recommendation.

**[Table 1]**

| primer | | sequence | SEQ ID: |
|---|---|---|---|
| 16S rDNA | 16S_V3_F | 5'- TCGTCGGCAGCGTCAGATGTGTATAAGAG ACAGCCTACGGGNGGCWGCAG-3' | 1 |
| | 16S_V4_R | 5'- GTCTCGTGGGCTCGGAGATGTGTATAAGA GACAGGACTACHVGGGTATCTAATCC-3 | 2 |

### 4. Microbial compositional analysis

Raw pyrosequencing reads were filtered, using MiSeq (Illumina, USA), according to raw pyro barcode and primer sequences obtained from a sequencer. High-quality sequencing reads were selected with a long read length of 300 bp or more and a Phred score of 20 points or more (accuracy of reference call > 99%). Operational Taxonomic Units (OTUs) were clustered using the sequence clustering algorithm CD-HIT. Thereafter, taxonomy assignment was performed on the 16S rDNA sequence database of GreenGenes 8.15.13 using UCLUST and QIIME, and taxonomy assignment at the genus level was performed on all 16S rDNA sequences based on sequence similarity. A bacterial composition at each level was made up of stacked bars. When the database did not have any sequence or the cluster could not be assigned at the genus level due to duplicate sequences, the classification table was assigned at the next higher level, as shown in parentheses.

### 5. Clinical study design

Study subjects were divided into four groups. The first group was a group of 239 patients under the age of 40 who visited Asan Medical Center in Seoul, and doctors diagnosed the patients with asthma and reversible airway obstruction (forced expiratory volume in one second (FEV1) after the use of a bronchodilator or surgery was increased by 15% or more from the reference value), regardless of each patient's smoking history. The second group is a group of 205 patients aged 40 years or older who visited Asan Medical Center, and doctors diagnosed the patients with COPD and irreversible airway obstruction (FEV1/forced vital capacity (FVC) after the use of a bronchodilator < 0.7) regardless of each patient's smoking history. The third group consisted of 324 histologically confirmed lung disease patients hospitalized in Dankook University Hospital in Cheonan. The fourth group consisted of 88 of a healthy control without any respiratory disease as a result of a health examination at the Asan Medical Center Health Screening & Promotion Center. This group did not include any individual without clinical data. This study was approved by the Research Ethics Committee of Asan Medical Center (approval number, IRB 2014-0360), and a consent to provide information was obtained from each participant.

### 6. ELISA

In order to measure the titers of anti-bacterial EV IgG of a human serum sample, 96-well plates were coated with 50 ng of bacterial EV overnight. In order to quantify the titers of anti-bacterial EV IgG, IgG1 and IgG4, the 96-well plates were coated with anti-human IgG, IgG1 and IgG4 antibodies (Abcam, Cambridge, UK) instead of the bacterial EV. The next day, wells coated with dust EV were blocked with PBS containing 5% skim milk, and a serum sample diluted 1:1,000 with 5% skim milk was added thereto. Anti-IgG, IgG1 and IgG4 (Abcam, Cambridge, UK) conjugated with horseradish peroxidase were used to detect antibodies, and were measured using a microplate reader.

When the sensitivity of IgG, IgG1 and IgG4 to dust EV exceeds 95% of the control, arbitrary criteria were defined as high anti-bacterial EV IgG, IgG1 and IgG4 in serum.

In addition, in order to evaluate the sensitivity of the analysis method, a limit of detection (LOD) and a limit of quantification (LOQ) were evaluated. The LODs of IgG, IgG1 and IgG4 were 0.011 µg/ml, 0.008 µg/ml and 0.006 µg/ml, respectively. Meanwhile, the LOQs of IgG, IgG1 and IgG4 were 0.027 µg/ml, 0.020 µg/ml and 0.015 µg/ml, respectively.

### 7. Statistical analysis

Significant differences between bacteria and bacterial EV in indoor dust were tested using the *Mann-Whitney U* test for continuous variables. Furthermore, Pearson correlation analysis was performed between the samples. In order to compare serum antibodies of patients with asthma, patients with chronic obstructive pulmonary disease, patients with lung cancer and healthy controls, a T-test was used. In order to develop a diagnostic model, the presence or absence of smoking was specified as a covariate, and a logistic regression analysis method was performed using biomarkers that showed a significant difference. P values of 0.05 or less were considered statistically significant. All statistical analyses were performed using R 3.4.1 version.

### Example 1. Metagenome of bacteria and bacterial EVs in indoor dust

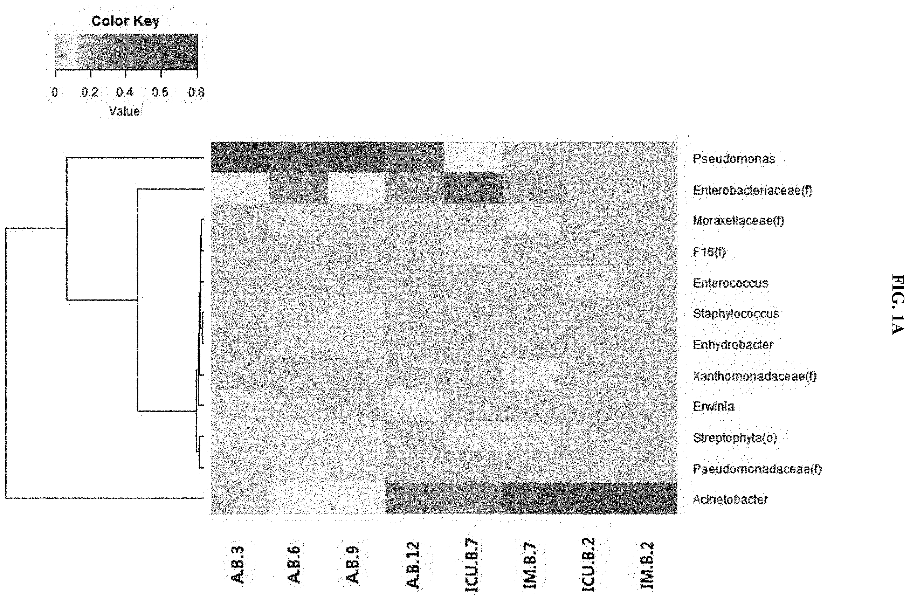

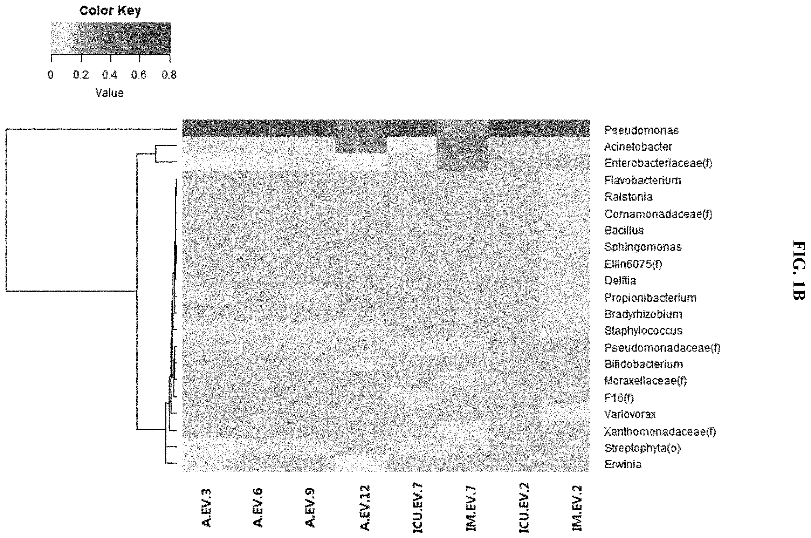

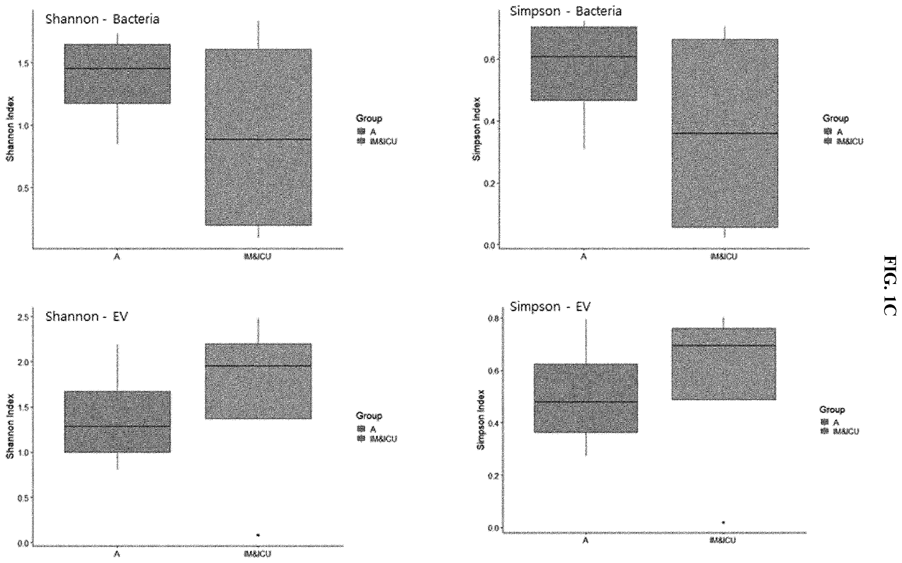

As a result of an analysis of bacteria and EVs which are indoor dust samples collected in apartment houses and hospitals, proteobacteria were the most abundant at the phylum level, and showed an average of 92.4% (SD5.3) and 81.2% at the lowest concentration. *Pseudomonas, Enterobacteriaceae* (f), and *Acinetobacter* were the most abundant at the genus level, and *Pseudomonas* and *Acinetobacter* were the most abundant in apartment houses and hospitals (FIG. 1A). *Pseudomonas* EV was the most abundant in hospitals and apartment houses (FIG. 1B). Further, in the diversity analysis, Shannon diversity index and Simpson index (comparative analysis of abundance and uniformity), *Pseudomonas* showed abundance at the bacterial level, but EV of *Pseudomonas* was not abundant, which did not show a statistically significant difference (FIG. 1C).

As a result of an analysis of indoor dust, bacteria and EVs in the same sample of apartment houses showed a higher correlation (mean r = 0.97) with each other than in hospitals (mean r = 0.30). Proteobacteria were the most abundant, with 94.9% (SD 2.2) at the phylum level and 90.1% (SD 2.9) at the EV level, although there was a slight difference depending on the time at which the dust sample was collected. *Pseudomonas, Enterobacteriaceae* (f), and *Acinetobacter* were above average at the genus level with 86.6% (SD 3.9) of their bacteria and 81.2% (SD 7.9) in EVs.

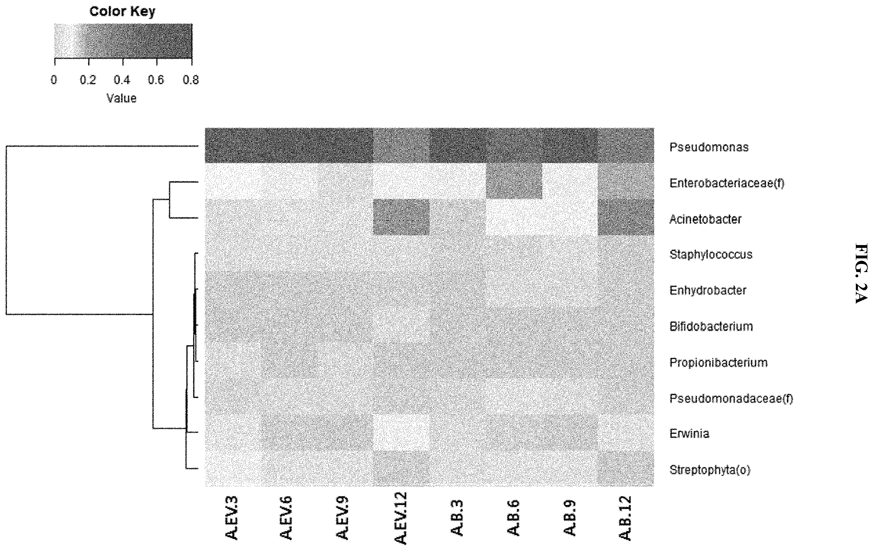

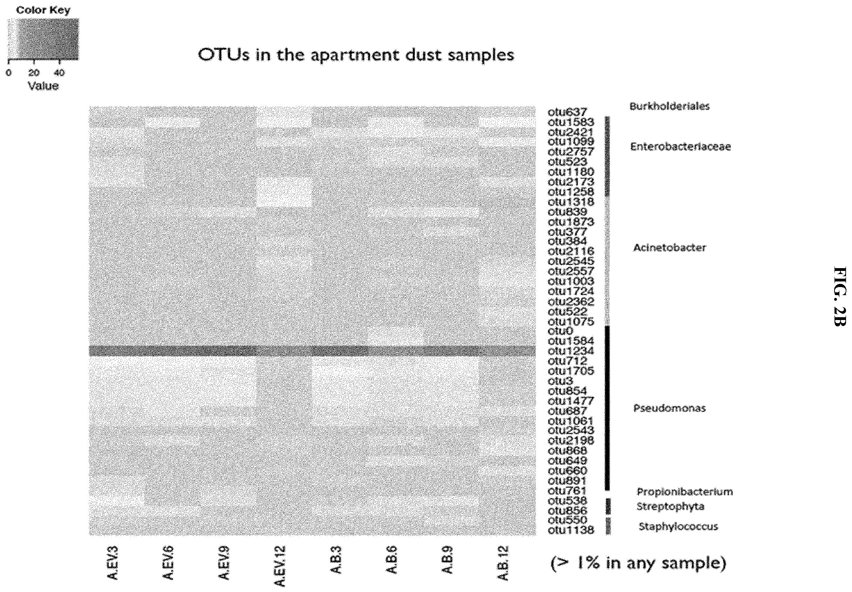

According to an OTU analysis for 1% or more of bacteria, it was shown that 16 OTUs belonged to *Pseudomonas,* 8 OTUs belonged to *Enterobacteriaceae* (f), 13 OTUs belonged to *Acinetobacter,* 2 OTUs belonged to *Streptophyta* (o) and *Staphylococcus,* and 1 OTU belonged to *Burholderiales*(o) and *Propinobacterium* (FIG. 2B).

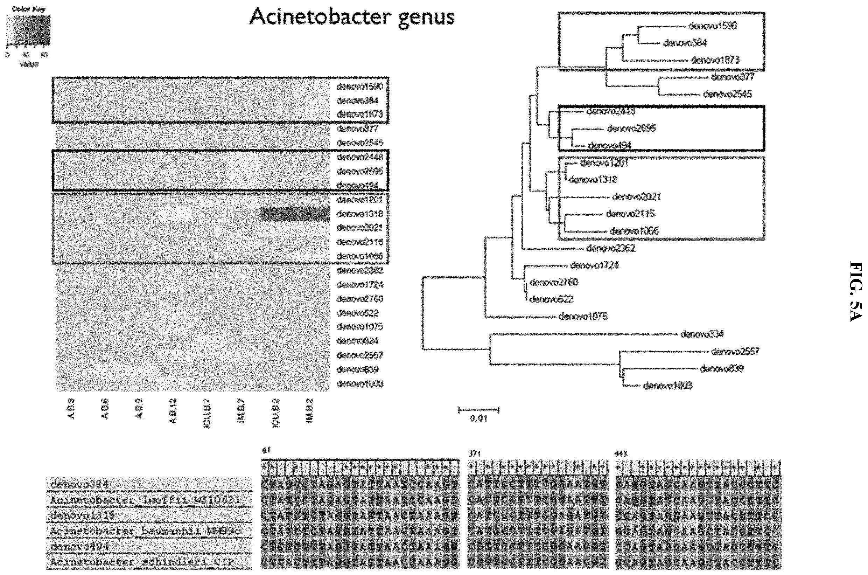

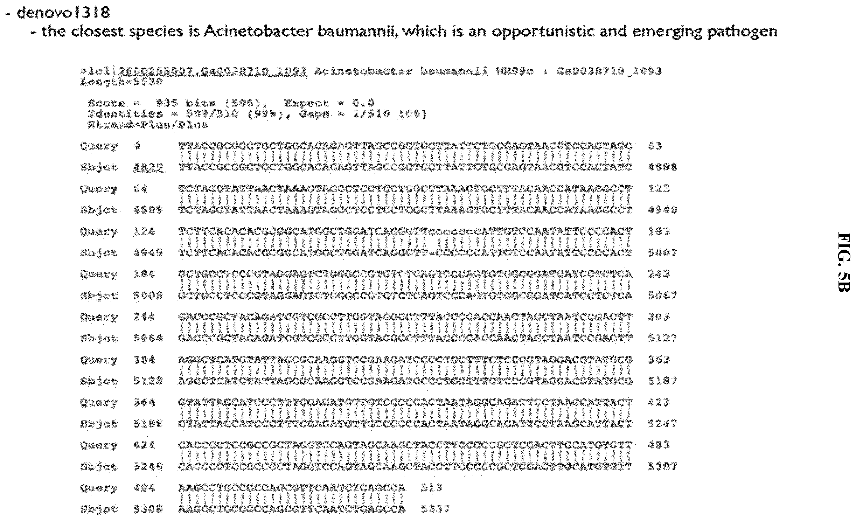

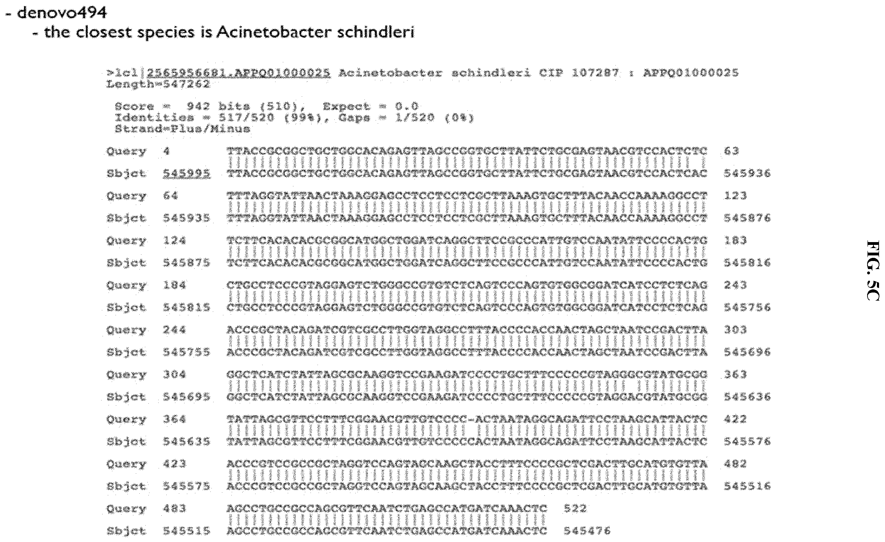

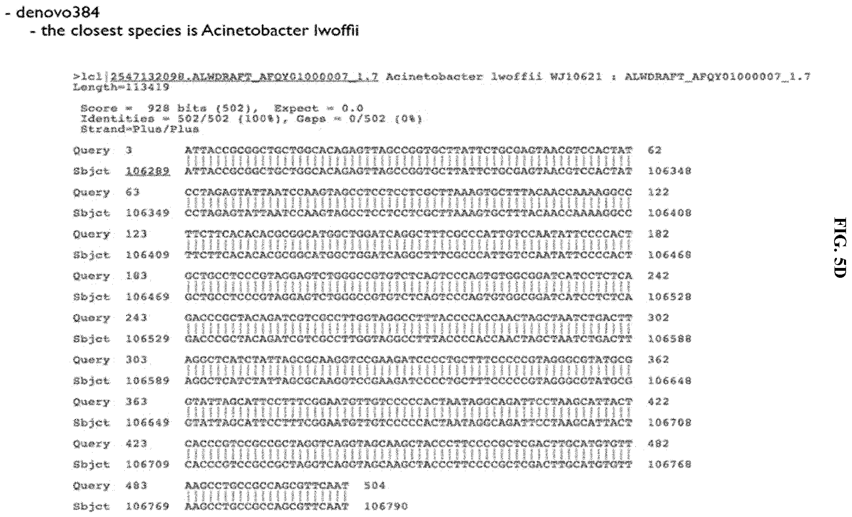

The most abundant species in the genus *Acinetobacter* were *Acinetobacter baumanii, Acinetobacter schindleri,* and *Acinetobacter lwoffii* (FIG. 5).

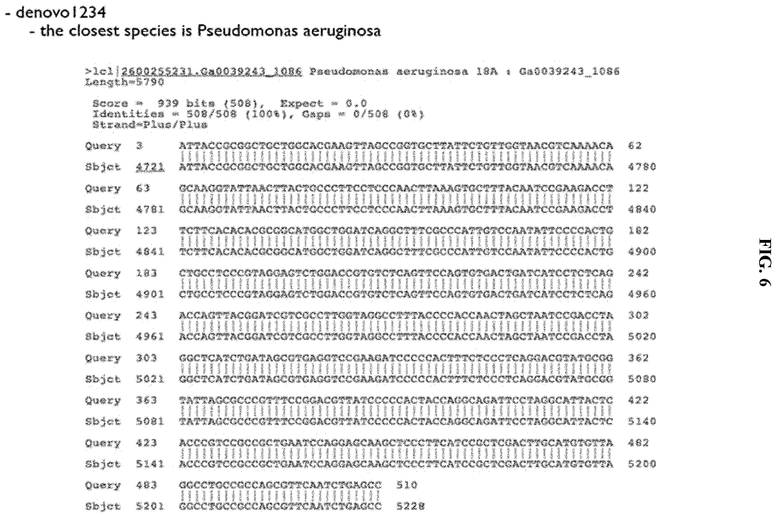

The most abundant species in the genus *Pseudomonas* was *Pseudomonas aeruginosa* (FIG. 6).

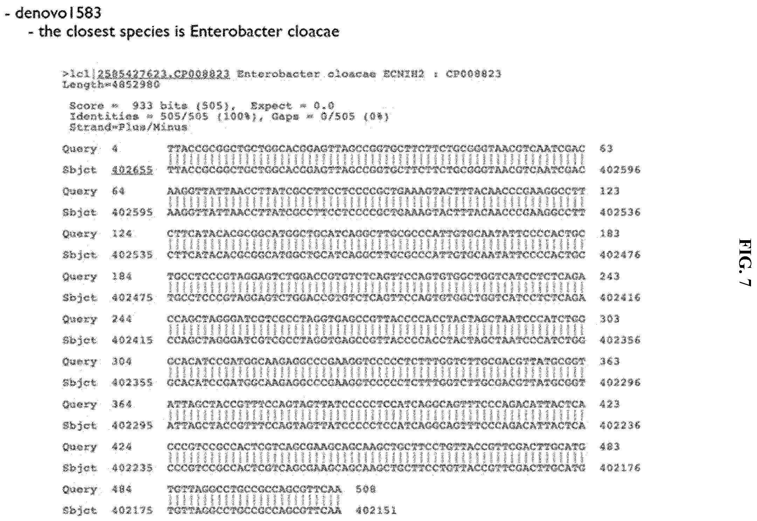

The most abundant species in the genus *Enterobacteriaceae* (f) was *Enterobacter cloacae* (FIG. 7).

### Example 2. Clinical study individual analysis

In this study, a total of 88 control subjects (49 males and 39 females), 239 patients with asthma (103 males and 136 females), 205 patients with COPD (196 males and 9 females), and 324 patients with lung cancer (274 males and 50 females) participated (Table 2). The average age of patients with asthma, COPD and lung cancer was significantly higher than that of the control (P < 0.001). In addition, patients with COPD and lung cancer were more likely to be male (P < 0.001) and smokers (P < 0.001) than the control, while patients with asthma had a similar sexual distribution and were less likely to smoke than the healthy control.

**[Table 2]**

| | | | | | **P-value** | | | |
|---|---|---|---|---|---|---|---|---|
| | **Healthy** | **Asthma** | **COPD** | **Lung cancer** | **Healthy vs. Asthma** | **Healthy vs. COPD** | **Healthy vs. Lung carter** | **COPD vs. Lung cancer** |
| N (Male / Female) | 88 (49/39) | 239 (103/136) | 205 (196/9) | 324 (274/50) | 0.0575 | <0.0001 | <0.0001 | 0.0002 |
| Age (Mean ± SD) | 50.8 ± 9.8 | 55.5± 14.5 | 66.4± 7 | 65.7± 9 | 0.0008 | <0.0001 | <0.0001 | 0.3044 |
| Smoking (0 / 1)* | 88 (45/43) | 239 (174/65) | 205 (0/205) | 324 (79/245) | 0.0004 | <0.0001 | <0.0001 | <0.0001 |

### Example 3. IgG comparative analysis of bacterial EVs using ELISA

According to the results of the metagenome analysis, an ELISA analysis was performed using serum anti-EV IgG, serum anti-EV IgG1, and serum anti-EV IgG4 targeting *Staphylococcus aureus (S. aureus), Acinetobacter baumannii (A. baumannii), Enterobacter cloacae (E. cloacae)* and *Pseudomonas aeruginosa (*P. aeruginosa*)*.

As illustrated in FIG. 3, as a result of comparative analysis of control - patients with asthma, *Enterobacter* EV IgG, *Pseudomonas* EV IgG, *Staphylococcus* EV IgG, and *Acinetobacter* EV IgG1 were significantly increased (p < 0.05).

As a result of comparative analysis of control-patients with COPD, EV IgG of *Acinetobacter, Enterobacter, Pseudomonas, and Staphylococcus* was significantly increased (p < 0.05). In particular, *Acinetobacter* EVIgG1 showed a significant difference of 1.42-fold and 1.66-fold between asthma and COPD samples, respectively.

As a result of comparative analysis of control-patients with lung cancer, all of IgG, IgG1, and IgG4 of *Enterobacter* and *Pseudomonas* EVs showed a significant increase (p < 0.05). In particular, *Acinetobacter* EV IgG1, *Enterobacter* EV IgG4, *and Staphylococcus* EV IgG4 showed a significant difference of 2-fold or more.

Furthermore, as a result of comparative analysis of patients with COPD-patients with lung cancer, IgG4 of *Enterobacter* EV and IgG, IgG1, and IgG4 of *Staphylococcus* EV showed a significant increase in patients with lung cancer, and *Staphylococcus* EV IgG4 and *Enterobacter* IgG4 showed a significant difference of 2-fold or more (p < 0.05) (see FIG. 3 and Table 3).

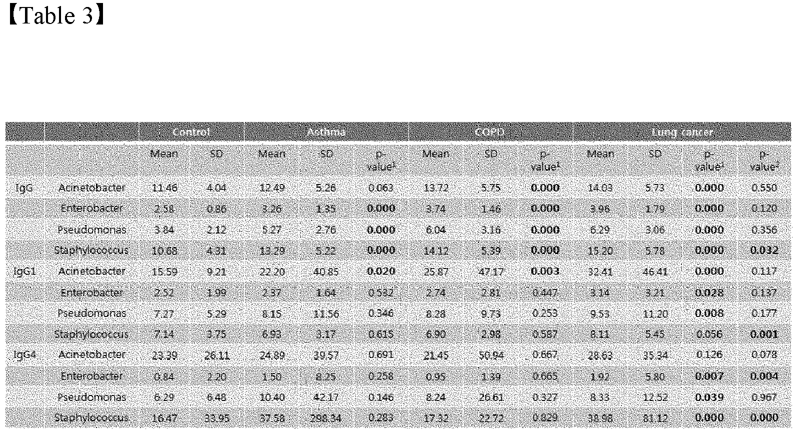

### Example 4. Diagnostic model based on IgG, IgG1 and IgG4

A diagnostic model was completed by a logistic regression analysis by adopting the presence and absence of smoking as a covariate, and an antibody that showed a significant difference in ELISA was selected as a biomarker.

The model was created by four methods and is as follows: i) use all markers that show a significant difference ii) add covariate factors for smoking or non-smoking of individuals while using all markers that show a significant difference iii) use only markers that show the most significant difference among IgG, IgG1, and IgG4 of each EV antibody, and iv) add covariate factors for smoking or non-smoking of individuals while using only markers that shows the most significant difference among IgG, IgG1, and IgG4 of each EV antibody.

Specific markers for each model are shown in the following Table 4.

**[Table 4]**

| **Model** | **Variable** |
|---|---|
| ADMi | y ~ aE IgG + aP IgG + aS IgG + aA IgG1 |
| ADMii | y ~ aE IgG + aP IgG + aS IgG + aA IgG1 + smoking |
| CDMi | y - aE IgG + aP IgG + aS IgG + aA IgG + aA IgG1 |
| CDMii | y ~ aE IgG + aP IgG + aS IgG + aA IgG + aA IgG1 - smoking |
| CDMiii | y ~ aE IgG + aP IgG + aS IgG + aA IgG |
| CDMiv | y - aE IgG + aP IgG + aS IgG + aA IgG + smoking |
| LDMi | y ~ aE IgG + aP IgG + aS IgG + aA IgG + aE IgG1 + aP IgG + aA IgG1 + aE IgG4 + aP IgG4 + aS IgG4 |
| LDMii | y ~ aE IgG + aP IgG + as IgG + aA IgG + aE IgG1 + aP IgG + aA IgG1 + aE IgG4 + aP IgG4 + aS IgG4 + smoking |
| LDMiii | y ~ aE IgG + aP IgG + aS IgG + aA IgG1 |
| LDMiv | γ ~ aE IgG + aP IgG + aS IgG + aA IgG1 + smoking |
| LDMCi | y ~ aS IgG + aS IgG1 + aE IgG4 + aS IgG4 |
| LDMCii | y ~ aS IgG + aS IgG1 + aE IgG4 + as IgG4 + smoking |
| LDMCiii | y ~ aE IG4 + aS IgG4 |
| LDMCiv | y ~ aE IG4 + aS IgG4 + smoking |

| | |
|---|---|
| aE: anti-*E*. *cloacae* EV / aP: anti-*P*. *aeruginosa* EV / aS: anti-*S*. *aureus* EV / aA: anti-*A*. *baumannii* EV | |

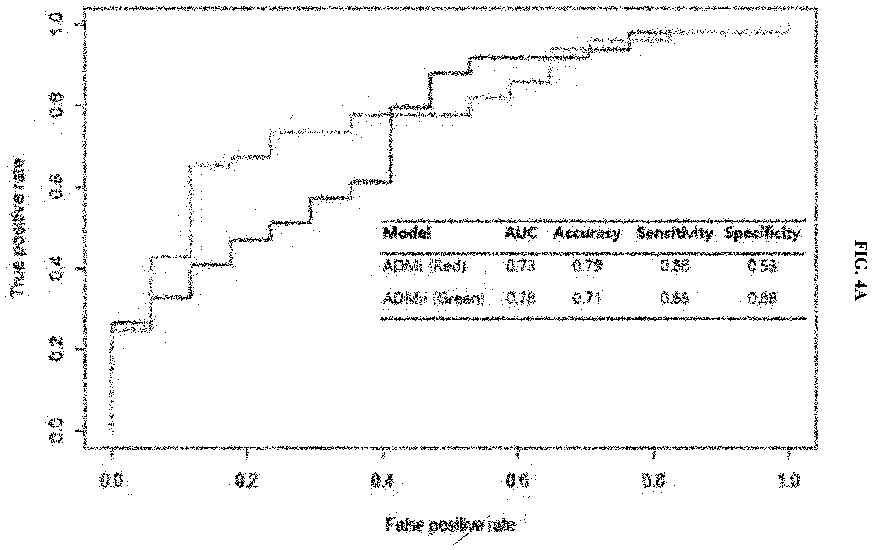

As illustrated in FIG. 4A, at least one significant marker was used for each applicable EV antibody in an asthma diagnostic model (ADM). AUC increased to AUC 0.78 when considering whether the individual previously smoked, and to AUC 0.73 when not considering whether the individual previously smoked.

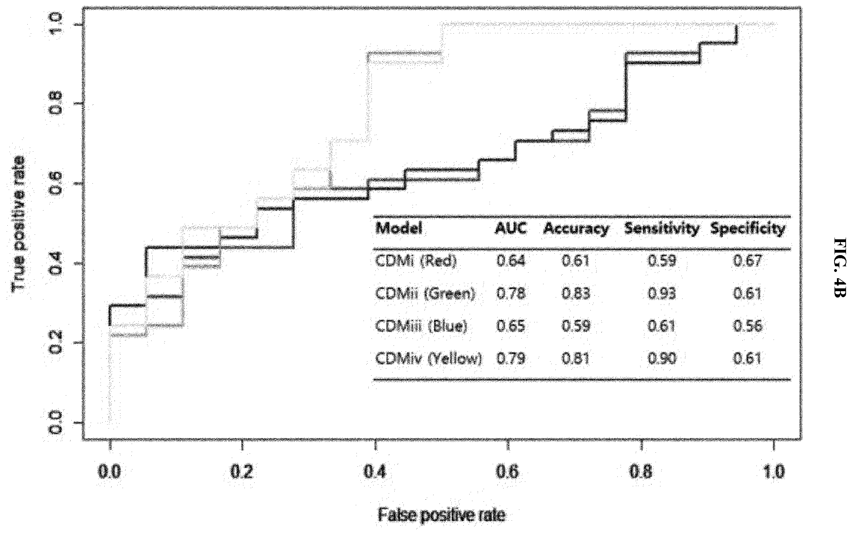

As illustrated in FIG. 4B, in a COPD diagnostic model (CDM), the i) and ii) models showed similar AUC results of 0.64 and 0.65, respectively. As a result of considering whether or not the subject smoked, it was found that the AUCs of i) and ii) considerably increased to 0.78 and 0.79, respectively.

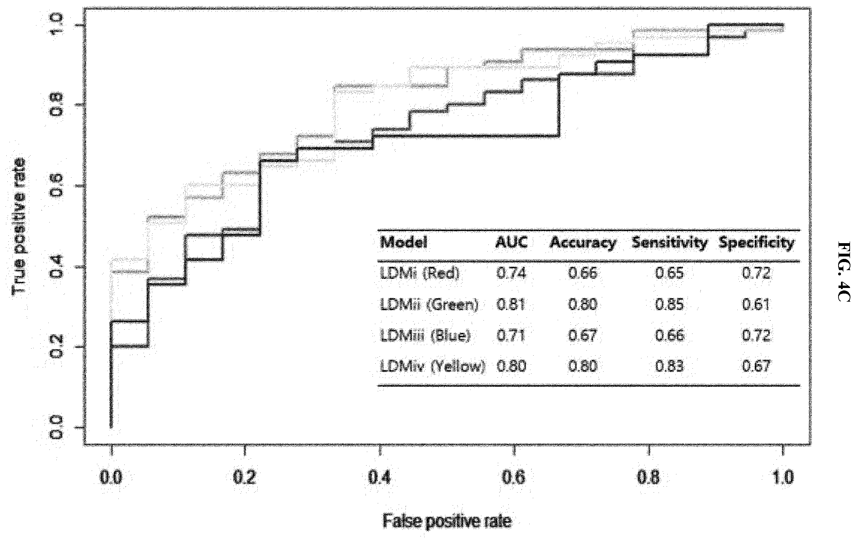

As illustrated in FIG. 4C, in a lung cancer diagnostic model (LDM) for the normal control, the i) model and iii) model were 0.74 and 0.71, respectively, confirming that the i) model showed more accurate results. As a result of considering the presence or absence of smoking, it was confirmed that the AUC of i) and iii) lung cancer diagnostic models for the normal control increased to 0.81 and 0.80, respectively.

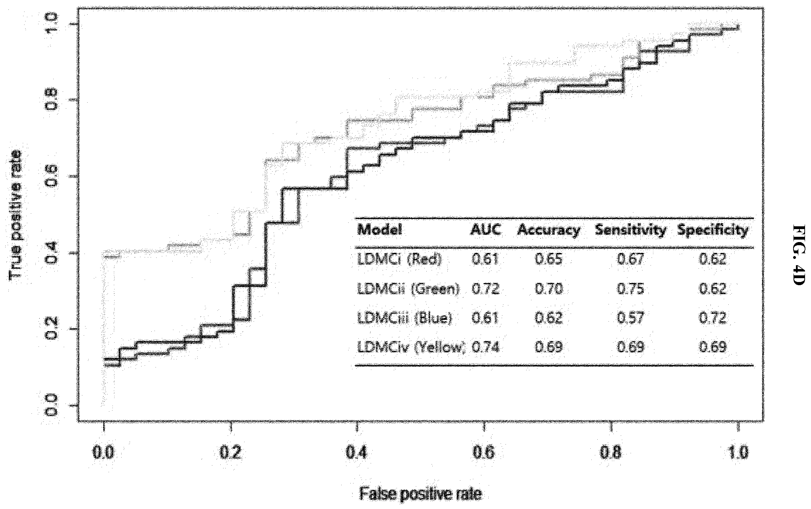

As illustrated in FIG. 4D, the AUC of both the i) model and ii) model was 0.61 in the lung cancer diagnostic model (LDM) for patients with COPD. As a result of considering whether or not the patient smoked, it was found that the AUC of each model considerably increased to 0.72 and 0.74.

As a result of summarizing the examples, in the present invention, it was confirmed that the serum IgG, IgG1, and IgG4 antibody titers of anti-bacterial EVs of indoor dust were significantly high in patients with asthma, COPD, and lung cancer, and the ELISA test could be used as a diagnostic tool based on anti-bacterial EV antibodies. Therefore, the present invention is expected to provide a new method for diagnosing a lung disease including asthma, COPD and lung cancer.

The above description of the present invention is provided only for illustrative purposes, and it will be understood by one of ordinary skill in the art to which the present invention pertains that the invention may be embodied in various modified forms without departing from the spirit or essential characteristics thereof. Thus, the embodiments described herein should be considered in an illustrative sense only and not for the purpose of limitation.

### [Industrial Applicability]

Since extracellular vesicles secreted from bacteria present in the environment can be absorbed into the body to have a direct effect on inflammatory responses and it is difficult to treat a lung disease such as asthma, chronic obstructive pulmonary disease, and lung cancer due to the difficulty in early diagnosis before symptoms thereof appear, it is possible to delay the onset time of the lung disease or prevent the onset of the lung disease through proper management by predicting the risk of developing the lung disease in advance through an antibody-based IgG, IgG1 and IgG4 level analysis of extracellular vesicles derived from bacteria using a human body-derived sample according to the present invention to diagnose and predict a risk group of the lung disease early, and it is possible to lower the incidence rate of a lung disease including asthma, chronic obstructive pulmonary disease and lung cancer and enhance a therapeutic effect due to the ability to diagnose the lung disease early even after the onset, so that the present invention has industrial applicability.

## Claims

1. A method for providing information for diagnosing a lung disease, the method comprising the following steps:
(a) obtaining a sample from a subject;
(b) measuring protein levels using antibodies against one or more proteins selected from the group consisting of extracellular vesicle IgG, extracellular vesicle IgG1 and extracellular vesicle IgG4 for the obtained sample; and
(c) comparing the protein levels with those of a control.

2. The method of claim 1, wherein the sample is blood.

3. The method of claim 2, wherein the blood is whole blood, serum, plasma, or blood mononuclear cells.

4. The method of claim 1, wherein the lung disease is one or more selected from the group consisting of asthma, chronic obstructive pulmonary disease, lung cancer, pneumonia, emphysema, chronic bronchitis, and pulmonary hypertension.

5. The method of claim 1, wherein the Step (c) comprises comparing an increase and decrease in levels of one or more selected from the group consisting of extracellular vesicle IgG, IgG1 and IgG4 derived from bacteria of one or more genera selected from the group consisting of *Staphylococcus, Acinetobacter, Enterobacter,* and *Pseudomonas.*

6. The method of claim 1, wherein the Step (c) comprises predicting that the risk of developing a lung disease is high when the levels of IgG of extracellular vesicles derived from one or more genus bacteria selected from the group consisting of *Staphylococcus, Acinetobacter, Enterobacter,* and *Pseudomonas* are increased

7. The method of claim 1, wherein the Step (c) comprises predicting that the risk of developing a lung disease is high when the levels of IgG1 of extracellular vesicles derived from one or more genus bacteria selected from the group consisting of *Staphylococcus, Acinetobacter, Enterobacter,* and *Pseudomonas* are increased

8. The method of claim 1, wherein the Step (c) comprises predicting that the risk of developing a lung disease is high when the levels of IgG4 of extracellular vesicles derived from one or more genus bacteria selected from the group consisting of *Staphylococcus, Acinetobacter, Enterobacter,* and *Pseudomonas* are increased

9. The method of claim 1, when the lung disease is asthma, wherein the Step (b) comprises measuring protein levels using an antibody against a protein of IgG of extracellular vesicles derived from bacteria of one or more genera selected from the group consisting of *Staphylococcus, Enterobacter,* and *Pseudomonas;* and an antibody against a protein of IgG1 of extracellular vesicles derived from the bacteria of the genus *Acinetobacter*

10. The method of claim 1, when the lung disease is COPD, wherein the Step (b) comprises measuring protein levels using an antibody against a protein of IgG of extracellular vesicles derived from bacteria of one or more genera selected from the group consisting of *Staphylococcus, Acinetobacter, Enterobacter* and *Pseudomonas;* and an antibody against a protein of IgG1 of extracellular vesicles derived from the bacteria of the genus *Acinetobacter.*

11. The method of claim 1, when the lung disease is lung cancer, wherein the Step (b) comprises measuring protein levels using an antibody against a protein of IgG of extracellular vesicles derived from bacteria of one or more genera selected from the group consisting of *Staphylococcus, Enterobacter* and *Pseudomonas;* and an antibody against a protein of IgG1 of extracellular vesicles derived from the bacteria of the genus *Acinetobacter.*

12. A composition for diagnosing a lung disease, comprising an agent which measures the levels of one or more proteins selected from the group consisting of extracellular vesicle IgG, extracellular vesicle IgG1 and extracellular vesicle IgG4.

13. The composition of claim 12, wherein the lung disease is one or more selected from the group consisting of asthma, chronic obstructive pulmonary disease, lung cancer, pneumonia, emphysema, chronic bronchitis, and pulmonary hypertension.

14. The composition of claim 12, wherein the extracellular vesicle is derived from bacteria of one or more genera selected from the group consisting of *Staphylococcus, Acinetobacter, Enterobacter* and *Pseudomonas.*

15. The composition of claim 12, wherein the agent is antibody that specifically binds to the protein.

16. A kit for diagnosing a lung disease comprising the composition according to claim 12.

17. A method for screening a therapeutic agent for a lung disease, the method comprising: administering a candidate material for treating a lung disease to a subject, and then measuring protein levels using antibodies against one or more proteins selected from the group consisting of extracellular vesicle IgG, extracellular vesicle IgG1 and extracellular vesicle IgG4 for a sample obtained from the subject.

18. A method for diagnosing a lung disease, the method comprising the following steps:
(a) obtaining a sample from a subject;
(b) measuring protein levels using antibodies against one or more proteins selected from the group consisting of extracellular vesicle IgG, extracellular vesicle IgG1 and extracellular vesicle IgG4 for the obtained sample; and
(c) comparing the protein levels with those of a control.

19. A method for providing information for predicting a risk for a lung disease, the method comprising the following steps:
(a) obtaining a sample from a subject;
(b) measuring protein levels using antibodies against one or more proteins selected from the group consisting of extracellular vesicle IgG, extracellular vesicle IgG1 and extracellular vesicle IgG4 for the obtained sample; and
(c) comparing the protein levels with those of a control.

20. Use of a composition comprising an agent which measures the levels of one or more proteins selected from the group consisting of extracellular vesicle IgG, extracellular vesicle IgG1 and extracellular vesicle IgG4 for diagnosing a lung disease.


<110>    MD Healthcare Inc.

<120>    Method for diagnosis of lung disease based on antibodies for
         microbial-derived vesicles

<130>    MPCT20-033

<150>    KR 10-2019-0111920
<151>    2019-09-10

<150>    KR 10-2020-0016035
<151>    2020-02-11

<160>    2

<170>    KoPatentIn 3.0

<210>    1
<211>    50
<212>    DNA
<213>    Artificial Sequence

<220>
<223>    16S_V3_F


<400>    1
tcgtcggcag cgtcagatgt gtataagaga cagcctacgg gnggcwgcag                50


<210>    2
<211>    55
<212>    DNA
<213>    Artificial Sequence

<220>
<223>    16S_V3_F


<400>    2
gtctcgtggg ctcggagatg tgtataagag acaggactac hvgggtatct aatcc          55